# EUROPEAN PATENT APPLICATION

(11) **EP 4 578 428 A1**
(43) Date of publication of application: **02.07.2025**
(21) Application number: 23901991.2
(22) Date of filing: 27.04.2023
(51) Int. Cl.: A61F 9/007

(54) **CATARACT SURGICAL INSTRUMENT**

(30) Priority: 15.12.2022 CN 202211613159
(71) Applicant: Rugao Wen Nuo Medical Technology Co., Ltd., Rugao Nantong, Jiangsu 226500 (CN)
(72) Inventor: CAI, Chengping, Wuxi, Jiangsu 214000 (CN); LIU, Shulong, Wuxi, Jiangsu 214000 (CN)
(74) Representative: Murgitroyd & Company
(86) International application number: PCT/CN2023/091066
(87) International publication number: WO 2024/124778

(57) **Abstract**

The present disclosure relates to the technical field of medical surgical instruments, and particularly, to a cataract surgical instrument. The cataract surgical instrument comprises a guide tube (3), a deformation member (2), and a net bag (1). An end portion of the guide tube (3) is provided with an opening (31) in communication with an inner cavity (35) of the guide tube (3). The deformation member (2) comprises a deformation part (21) and a connecting part (22) that is connected to the deformation part (21). The connecting part (22) is arranged through the inner cavity (35) and can move in the axial direction of the guide tube (3). The net bag (1) is connected to the deformation part (21). The connecting part (22) drives the deformation part (21) and the net bag (1) to move in the axial direction of the guide tube (3), such that the deformation part (21) and the net bag (1) retract into the inner cavity (35) or expand out of the inner cavity (35). By mechanically and centripetally cutting the crystalline lens, the cataract surgical instrument causes no thermal damage to intraocular tissues and is less prone to damaging the posterior capsule, zonules, and corneal endothelium. The instrument also features short operation duration, high efficiency, small surgical incisions, minimal discomfort, rapid recovery, ease to operate, and cost-efficiency, and avoids various disadvantages associated with ultrasonic vibration.

## Description

### TECHNICAL FIELD

The present disclosure relates to the technical field of medical surgical instruments, and particularly relates to a cataract surgical instrument.

### BACKGROUND

Cataract refers to a clouding of a lens of eye, which changes from transparent to opacity due to aging, physical and chemical damage, inflammation and other reasons. The clouding obstructs light from entering the eye, thereby impairing vision. Cataract is a leading cause of blindness. Common surgical treatments for cataracts include traditional extracapsular cataract extraction, extracapsular cataract extraction with small incision and manual chopping of nucleus, phacoemulsification with intraocular lens implantation, and femtosecond laser-assisted cataract surgery.

Compared with intracapsular cataract extraction, traditional extracapsular cataract extraction has the following advantages: the surgery is performed under a surgical microscope using microsurgical instruments, and surgical techniques have been greatly improved, creating favorable conditions for intraocular lens implantation. However, it also has the following disadvantages: a surgical incision is relatively large, the surgery lasts for a long period of time, and postoperative recovery is slower. Due to the large incision and significant astigmatism, the postoperative visual recovery is poor, and a patient needs to be hospitalized.

Extracapsular cataract extraction with small incision and manual chopping of nucleus is a surgery with a surgical incision size between that of traditional large-incision cataract surgery and minimally invasive cataract phacoemulsification surgery. However, compared with minimally invasive cataract phacoemulsification surgery, it has larger incision size, resulting in greater astigmatism, slower postoperative recovery, and long operation duration. Specifically, the incision size is generally about 5.5 mm, and the surgery usually lasts about 15-30 minutes. Moreover, it may result in higher incidence of complications, such as corneal endothelial injury leading to corneal edema; iris injury leading to anterior chamber hemorrhage or root detachment; posterior capsule rupture, which may prevent intraocular lens implantation and cause expulsive choroidal hemorrhage, and other postoperative complications, and a most serious complication is intraocular infection. This type of surgery is suitable for areas with underdeveloped economies or regions without access to phacoemulsification equipment for cataracts.

Phacoemulsification with intraocular lens implantation has the following advantages: small surgical incision, mild surgical response, minimal postoperative astigmatism, shorter operating time, and no hospitalization required. Specifically, the traditional surgical incision size is approximately 12 mm, while a size of the phacoemulsification incision is smaller than 3 mm; the incision healing is fast, and visual recovery is faster and better; the postoperative astigmatism is minimal and easier to correct or control, providing greater safety and stability; the surgery is usually completed within 15 minutes; patients can go home immediately after surgery, and the surgery does not require the cataract to be fully mature; and the patients are even painless, and has less surgical and postoperative complications. However, it also has some unavoidable disadvantages due to physical properties of ultrasound, specifically including:
1. uncertainty in a range of ultrasound action: during cataract phacoemulsification, a range of ultrasound action is impossible to be perceived by a surgeon, and the range of ultrasound action is not limited to an area around an opening of phacoemulsification needle tip, but also extends along an energy transmission path. During the surgery, fragmentation and emulsification of the cataract observed by the surgeon are merely results, while microscopic process, ultrasound itself, the range and mode of range of ultrasound action cannot be observed during the surgery, increasing a surgical risk;
2. non-selectivity of ultrasound action: it not only affects cataract, and energy transmitted by the ultrasound, including resulting impact and cavitation effects, cause physical and chemical changes in other intraocular tissues as well, thereby easily damaging the eye;
3. invisibility of ultrasound effects: most of the physical and chemical changes in other tissues in the eye occur at a molecular level, which is a microscopic process that cannot be observed by people. Only outcomes of the changes can be seen. In fact, outcomes of the changes are usually delayed, and degree of the changes is associated with an amount of ultrasound energy released during the surgery;
4. cavitation effect of ultrasound: repulsive force generated by ultrasound and bubbles created by cavitation will affect the fluid dynamics performance during cataract aspiration;
5. mechanical effect of ultrasound (mechanical effect caused by reflection when the ultrasound propagates in a medium): ultrasound can trigger several physiological responses. Specifically, substances in tissue cells produce the effect of cell message, also known as "internal massage", caused by cell plasma flow, cells rotate and rub due to fine massage of ultrasound, which is an unique feature of ultrasound treatment, and can change the permeability of cell membrane, stimulate a diffusion process of semi-permeable cell membrane, promote metabolism, accelerate blood and lymph circulation, improve cell ischemia and hypoxia, enhance tissue nutrition, change protein synthesis rate, improve regeneration function, and the like, such that an internal structure of cells change, cellular function is accordingly changed, and rigid connective tissue is stretched and softened; and
6. thermal effect of ultrasound: standing waves are formed when ultrasound waves reflect at a boundary of different tissues, and mutual motion of molecule generates friction and heat, which will easily damage an eyeball.

Femtosecond laser-assisted cataract surgery completes the most challenging step in traditional surgery with computer-controlled precision, which uses femtosecond laser pulses to create incisions in lens capsule, crystalline lens, and cornea, such that the surgery can be tailored to meet specific requirements of the surgeon, bringing the precision of surgery to a new level. Compared with traditional phacoemulsification surgery, the femtosecond laser-assisted surgery can reduce phacoemulsification energy by 43% and phacoemulsification time by 51%. Since the incisions are made without great force, possible damage to the eyes by the surgeon and instruments is eliminated. Moreover, the entire process of surgery is completely controlled by computer digitalization and completed by femtosecond laser, which is accurate and reduces a standard deviation of the surgeon's operation. A microscopic level of the surgery is precise and predictable; with accurate, reliable, predictable and repeatable incision creation. However, a biggest disadvantage of the surgery is its high cost, which limits its accessibility and general applicability.

Since the above surgeries have various disadvantages, it is necessary to design a cataract surgical instrument that combines the advantages of the above surgeries, such as small incision size (less than 3 mm), short operation duration, cost-effective and practical, broad applicability, and the like. In addition, the cataract surgical instrument should avoid various damages, and overcome various disadvantages caused by ultrasonic vibration.

### SUMMARY

In order to solve the above problems, the present disclosure provides a cataract surgical instrument. When in use, the instrument can create a small surgical incision of less than 3 mm, without increasing a number of existing incisions. The instrument does not require the use of ultrasound or laser, thereby avoiding various disadvantages of ultrasonic vibration. It is less prone to damaging posterior capsule, zonules, or corneal endothelium. The instrument also features short operation duration, high efficiency, small surgical incisions, minimal discomfort, rapid recovery, ease to operate, and cost-effective, and widely applicable without increasing financial burden of the patient.

An objective of the present disclosure is to provide a cataract surgical instrument, including:
a guide tube, and an end portion of the guide tube is provided with an opening in communication with an inner cavity of the guide tube;
a deformation member, the deformation member includes a deformation part and a connecting part that is connected to the deformation part, and the connecting part is arranged through the inner cavity and can move in an axial direction of the guide tube; and
a net bag, and the net bag is connected to the deformation part;
where the connecting part drives the deformation part and the net bag to move in the axial direction of the guide tube, such that the deformation part and the net bag retract into the inner cavity or expand out of the inner cavity.

In one embodiment of the present disclosure, the cataract surgical instrument further includes a push rod, the push rod is connected to the connecting part, and is configured to drive the connecting part to move in the axial direction of the guide tube.

In one embodiment of the present disclosure, the cataract surgical instrument further includes an outer tube, the guide tube is connected within the outer tube, and the push rod is arranged through both the outer tube and the guide tube and is connected to the connecting part.

In one embodiment of the present disclosure, the guide tube includes a first tube section and a second tube section that is connected to the first tube section, the opening is located at an end of the first tube section away from the second tube section, an outer wall of the second tube section is provided with at least one protrusion, an end portion of the outer tube is provided with a slot that matches the protrusion, and the guide tube is assembled in the slot.

In one embodiment of the present disclosure, an interior of the outer tube is sequentially provided with a first cavity, a second cavity, and a third cavity, a cross section of the second cavity is smaller than cross sections of the first cavity and the third cavity, and the guide tube is assembled in the first cavity.

In one embodiment of the present disclosure, the push rod includes a first rod body and a second rod body, a cross section of the first rod body is smaller than a cross section of the second rod body, the second rod body is arranged through the third cavity, and the first rod body is arranged through the first cavity and the second cavity and is fixedly connected to the connecting part.

In one embodiment of the present disclosure, a push handle is arranged at an end of the second rod body away from the first rod body, and the push handle is located outside the outer tube, and a plug is disposed between the push handle and the outer tube.

In one embodiment of the present disclosure, a size of the deformation part is larger than a size of the inner cavity.

In one embodiment of the present disclosure, the deformation part is a ring structure formed by winding a long strip, the ring structure is an open-ring structure or a closed-ring structure, the long strip is made from a shape memory material, a height of the long strip is 0.01-2 mm, and a thickness of the long strip is 0.01-2 mm.

In one embodiment of the present disclosure, the net bag is a bag-like structure with an aperture, the net bag is woven by a plurality of ultrafine filaments and formed with a plurality of mesh holes, and the aperture of the net bag is connected to the long strip.

### Beneficial Effects

(1) Adopting a mechanical centripetal cutting method of a lens, the present disclosure mechanically and centripetally cuts the lens, without causing thermal damage to intraocular tissues. The method is less prone to damaging the posterior capsule, zonules, and corneal endothelium, and features short operation duration, high efficiency, small surgical incisions, minimal discomfort, rapid recovery, ease to operate, and cost-efficiency, and widely applicable without increasing financial burden of the patient; it can create a small surgical incision without increasing a number of existing incisions, and it does not require ultrasound or laser, thereby avoiding various disadvantages of ultrasonic vibration.
(2) By using the telescopic deformation part and the net bag, the present disclosure can easily enter and exit from an interior of an eyeball through a small surgical incision. When the deformation part enters the interior of the eyeball, the deformation part drives the net bag to open and wrap around a cloudy lens nucleus, and the deformation part drives the net bag to contract when the connecting part is pulled, thereby automatically tightening and cutting the lens nucleus, and completely breaking the lens nucleus apart. The present disclosure adopts a mechanical centripetal cutting method of a lens, the surgery can be completed through a very small surgical incision without increasing a number of existing incisions, thereby significantly reducing injury to the eyeball of a patient and shortening the postoperative recovery time, thereby having fewer surgical and postoperative complications. Therefore, the present disclosure is cost-effective and widely applicable.
(3) The deformation part in the present disclosure is a ring structure, the ring structure is an open-ring structure or a closed-ring structure, and exhibits superelasticity, and the material thereof has memory properties, such that it can recover its original shape immediately after deformation, and can smoothly enter and exit from the eyeball freely through a small surgical incision.
(4) The net bag in the present disclosure is a bag-like structure with an aperture, the net bag is woven by a plurality of ultrafine filaments and formed with a plurality of mesh holes, and the aperture of the net bag is connected to the long strip. By controlling a number of ultrafine filaments and a size of mesh spacing, a particle size of the fragmented lens nucleus can be controlled, and strength of the ultrafine filaments can cut the lens nucleus of any hardness.

### BRIEF DESCRIPTION OF THE DRAWINGS

FIG. 1 is a perspective view of a cataract surgical instrument according to the present disclosure.
FIG. 2 is an exploded view of a cataract surgical instrument according to the present disclosure.
FIG. 3 is a front view of a cataract surgical instrument according to the present disclosure.
FIG. 4 is a sectional view of a cataract surgical instrument according to the present disclosure.
Fig. 5 is an assembly view of a guide tube and an outer tube according to the present disclosure.
FIG. 6 is a sectional view of an outer tube according to the present disclosure.
FIG. 7 is a front view of a push rod according to the present disclosure.
FIG. 8 is a top view of a deformation member according to the present disclosure.
FIG. 9 is a perspective view of a guide tube according to the present disclosure.
FIG. 10 is a sectional view of a guide tube according to the present disclosure.

Reference numerals in the accompanying drawings: 1. net bag; 2. deformation member; 21. deformation part; 22. connecting part; 3. guide tube; 31. opening; 32. first tube section; 33. second tube section; 34. protrusion; 35. inner cavity; 4. outer tube; 41. first cavity; 42. second cavity; 43. third cavity; 44. slot; 5. plug; 6. push rod; 61. first rod body 62. second rod body; and 63. push handle.

### DETAILED DESCRIPTIONS OF THE EMBODIMENTS

The technical solutions of embodiments of the present disclosure will be described below clearly and comprehensively in conjunction with the accompanying drawings of the embodiments of the present disclosure. Apparently, the embodiments described are some embodiments rather than all embodiments of the present disclosure. Based on the embodiments of the present disclosure, all other embodiments acquired by those of ordinary skill in the art without making creative efforts fall within the scope of protection of the present disclosure.

In the present disclosure, unless otherwise explicitly specified and defined, the terms "connecting", "connection", "fixing", etc. should be understood in a broad sense, for example, they may be a fixed connection, a detachable connection, or an integrated connection; may be a mechanical connection, or an electrical connection; may be a direct connection, or an indirect connection via an intermediate medium; and may be communication inside two elements, or an interactive relation between two elements. For those of ordinarily skilled in the art, specific meanings of the above terms in the present disclosure could be understood according to specific circumstances.

In the present disclosure, unless otherwise explicitly specified and defined, a first feature being "above" or "below" a second feature may include the first and second features being in direct contact or that the first and second features being not in direct contact but being in contact by means of additional features between the first and second features. Further, a first feature being "over," "above," and "on" a second feature may mean that the first feature is directly above and obliquely above the second feature, or simply mean that the first feature is higher than the second feature. A first feature being "below," "under," and "beneath" a second feature may mean that the first feature is directly below or diagonally below the second feature, or simply mean that the first feature is lower than the second feature.

### Embodiment 1

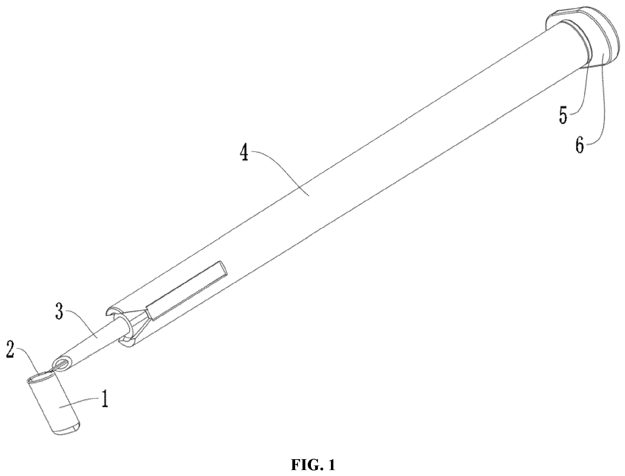

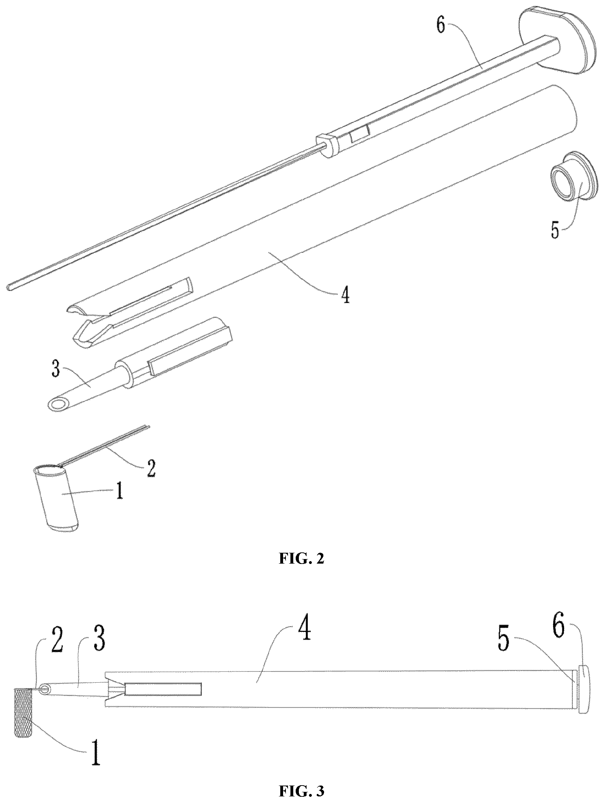

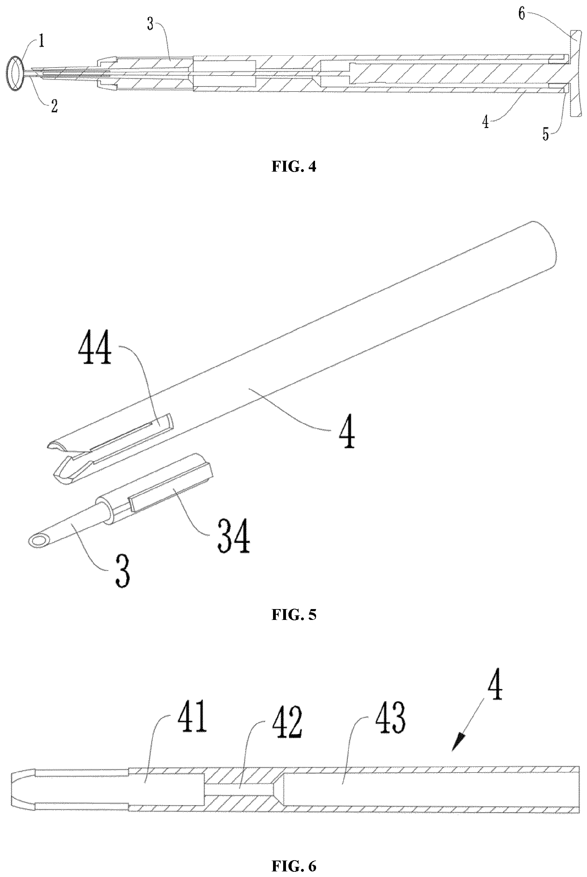

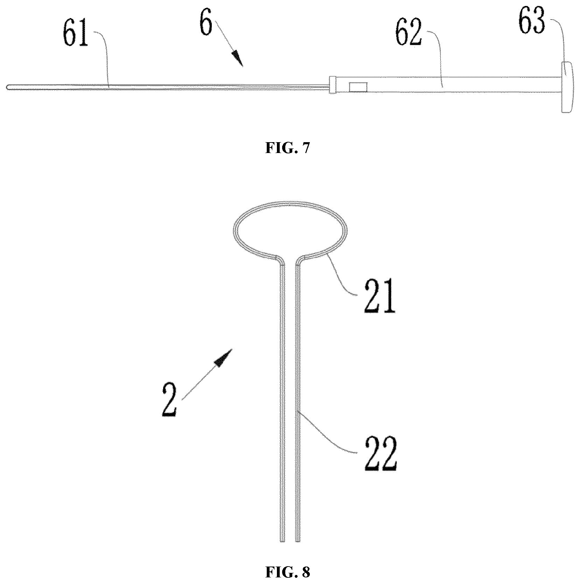

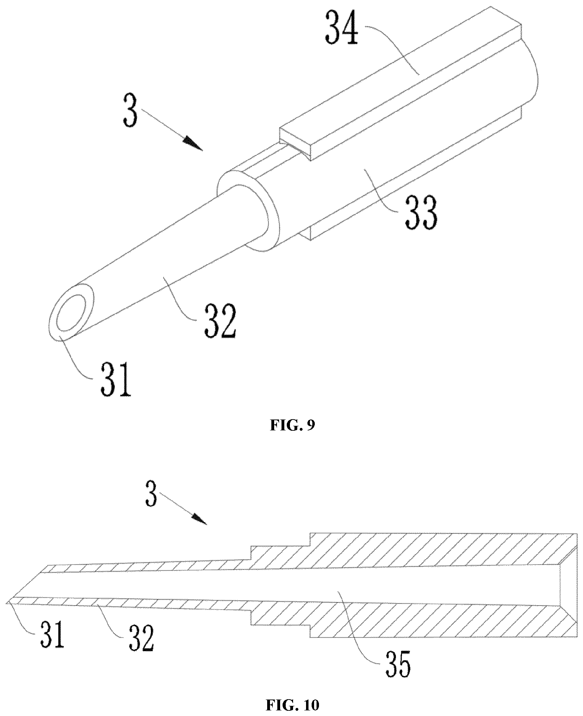

As shown in FIGs. 1-10, this embodiment provides a cataract surgical instrument, including a net bag 1, a deformation member 2, and a guide tube 3, where an end portion of the guide tube 3 is provided with an opening 31 in communication with an inner cavity 35 thereof; the deformation member 2 includes a deformation part 21 and a connecting part 22 that is connected to the deformation part 21, and the connecting part 22 is arranged through the inner cavity 35 and can move in an axial direction of the guide tube 3; the net bag 1 is connected to the deformation part 21; where the connecting part 22 drives the deformation part 21 and the net bag 1 to move in the axial direction of the guide tube 3, such that the deformation part 21 and the net bag 1 retract into the inner cavity 35 or expand out of the inner cavity 35. The telescopic deformation part 21 and the net bag 1 can easily enter and exit from an interior of an eyeball through a small surgical incision. When the deformation part 21 enters the interior of the eyeball, the deformation part 21 drives the net bag 1 to open and wrap around a cloudy lens nucleus, and the deformation part 21 drives the net bag 1 to contract when the connecting part 22 is pulled, thereby automatically tightening and cutting the lens nucleus, and completely breaking the lens nucleus apart. The present disclosure adopts a mechanical centripetal cutting method of a lens, which generates almost no heat and causes no thermal damage to intraocular tissue; the surgery can be completed through a very small surgical incision without increasing a number of existing incisions, thereby significantly reducing injury to the eyeball of a patient and shortening the postoperative recovery time; further, the method is less prone to damaging posterior capsule, zonules, or corneal endothelium, thus fewer surgical and postoperative complications. Moreover, the method is easy to learn, involves short operation duration, is efficient and less pain for the patient, and leads to rapid recovery; it is simple to operate, convenient, cost-effective, and widely applicable without increasing financial burden of the patient; and it does not require ultrasound or laser, thereby avoiding various disadvantages of ultrasonic vibration.

Optionally, the cataract surgical instrument further includes a push rod 6, the push rod 6 is connected to the connecting part 22, and is configured to drive the connecting part 22 to move in the axial direction of the guide tube 3.

Optionally, the cataract surgical instrument further includes an outer tube 4, the guide tube 3 is connected within the outer tube 4, and the push rod 6 is arranged through both the outer tube 4 and the guide tube 3 and is connected to the connecting part 22.

Optionally, the guide tube 3 includes a first tube section 32 and a second tube section 33 that is connected to the first tube section 32, the opening 31 is located at an end of the first tube section 32 away from the second tube section 33, an outer wall of the second tube section 33 is provided with at least one protrusion 34, an end portion of the outer tube 4 is provided with a slot 44 that matches the protrusion 34, and the guide tube 3 is assembled in the slot 44.

Optionally, an interior of the outer tube 4 is sequentially provided with a first cavity 41, a second cavity 42, and a third cavity 43, a cross section of the second cavity 42 is smaller than cross sections of the first cavity 41 and the third cavity 43, and the guide tube 3 is assembled in the first cavity 41.

Optionally, the push rod 6 includes a first rod body 61 and a second rod body 62, a cross section of the first rod body 61 is smaller than a cross section of the second rod body 62, the second rod body 62 is arranged through the third cavity 43, and the first rod body 61 is arranged through the first cavity 41 and the second cavity 42 and is fixedly connected to the connecting part 22. Further, the cross section of the second cavity 42 is larger than that of the first rod body 61, such that the first rod body 61 can pass through the second cavity 42, and move in the axial direction the guide tube 3 in the second cavity 42.

It can be seen from the above description that the guide tube 3 is located inside the first cavity 41, the second rod body 62 is located inside the third cavity 43, and the second cavity 42 with a smaller cross section plays a limiting role, such that the second rod body 62 of the push rod 6 can move only in the third cavity 43, and the deformation part 21 and the net bag 1 connected to the deformation part 21 can move only inside the inner cavity 35 of the guide tube 3, thereby limiting axial displacement of the deformation part 21 and the net bag 1, and preventing the deformation part 21 and the net bag 1 from being pulled out of the outer tube 4 by the push rod 6.

Optionally, a push handle 63 is arranged at an end of the second rod body 62 away from the first rod body 61, the push handle 63 facilitates pushing and pulling of the push rod 6 for convenient operation; and the push handle 63 is located outside the outer tube 4, and a plug 5 is disposed between the push handle and the outer tube 4. Optionally, a section of protrusion is formed on an end of the second rod body 62 close to the first rod body 61, a cross section of the section of protrusion is larger than that of an inner cavity of the plug 5, and the plug 5 plays a limiting role, such that the second rod body 62 of the push rod 6 can move only in the third cavity 43, an extreme position of the push rod 6 being pulled out is a position that the protrusion on the second rod body 62 abuts against the plug 5, which can effectively prevent the push rod 6 from being pulled out of the outer tube 4.

Optionally, a size of the deformation part 21 is larger than a size of the inner cavity 35, the deformation part 21 is in a deformed state when being located inside the inner cavity 35, and the deformation part 21 returns to its original shape when being located outside the inner cavity 35. Specifically, an original shape of the deformation part 21 is elliptical, circular, flattened, or other shapes, which is not limited herein. Those skilled in the art can modify the shape of the deformation part 21 according to use requirements of the deformation part 21; and when deformation part 21 deforms, the shape of the deformation part is elongated, which is determined by a shape of the inner cavity 35.

Optionally, the deformation part 21 is a ring structure formed by winding a long strip, the ring structure is an open-ring structure or a closed-ring structure, the long strip is made from a shape memory material, with a height of 0.01-2 mm and a thickness of 0.01-2 mm. Specifically, a depth of a ring wall of the deformation part 21 with the ring structure, that is, the height of the long strip, is 0.01-2 mm, and a thickness of the ring wall that is, the thickness of the long strip, is 0.01-2 mm. The setting of the depth and thickness of the ring wall can minimize trauma to the surgical incision and enable relatively free entry and exit. The deformation part 21 exhibits superelasticity, and the material thereof has memory properties, such that it can recover its original shape immediately after deformation, and can smoothly enter and exit from the eyeball freely through a 3 mm surgical incision.

Optionally, the net bag 1 is a bag-like structure with an aperture, which is woven by a plurality of ultrafine filaments and formed with a plurality of mesh holes. Optionally, a mesh diameter is 0.01-4 mm, and preferably 0.03 mm. The aperture of the net bag 1 is connected to the long strip. By controlling a number of ultrafine filaments and a size of mesh spacing, a particle size of the fragmented lens nucleus can be controlled, and strength of the ultrafine filaments can cut the lens nucleus of any hardness.

Preferably, the characteristics of the lens nucleus are that the nucleus becomes harder toward a center thereof, the lens has a biconvex shape, with an anterior-posterior diameter of 4-5 mm, a diameter of 9 mm, an anterior curvature radius of 10 mm, and a posterior curvature radius of 6 mm; and an anterior surface is slightly flatter than an posterior surface, the anterior surface is about 3.6 mm away from a corneal vertex, the lens is composed of a plurality of layers of materials with different refractive indices, and the closer to a center, the denser it becomes optically. A refractive index gradient is available from an anterior pole to a posterior pole, and from a center to an equator. A hardness of the lens gradually increases from a periphery to a center, forming a gradient, with the nucleus becoming harder as it approaches the center. Moreover, since cortex around the lens nucleus is removed, the periphery and anterior and posterior surfaces of the lens nucleus are relatively soft, an overall size thereof is reduced. Therefore, in order to wrap the lens nucleus, the deformation part 21 in this embodiment is preferably elliptical, a longitudinal length thereof is preferably 6.6 mm, and a horizontal length thereof is preferably 3.6 mm.

Working principle of the present disclosure are is as follows:
1. The push rod is pulled first to retract the deformation part and the net bag into the inner cavity of the guide tube, the instrument is held manually to have the incision at a front end of the guide tube into an eyeball through an established 3 mm surgical incision. The incision at the end of the guide tube can facilitate easy entry and exit of the guide tube from the eyeball.
2. The push rod is then pushed have the deformation part pushed out of from the inner cavity of the guide tube, the deformation part is pushed into the eyeball and returns to its memory shape and expands, such that the deformation part and the net bag are connected to the deformation part to extend inside the eyeball.
3. The instrument is rotated, an auxiliary tool is used to block the lens nucleus from rotating, such that the ring deformation part wraps around the cloudy lens nucleus; in which case, the push rod is pulled to automatically tighten the deformation part and the net bag connected to the deformation part, thereby cutting the lens nucleus. The ultrafine filaments in the net bag break the hard and cloudy lens nucleus into pieces small enough to pass through the net holes, ensuring complete breaking; and retract the deformation part and the net bag are then retracted into the inner cavity of the guide tube, the surgical instrument is finally removed, and the completely broken cloudy matter can be flushed and cleaned with a rinsing tool.

Adopting a mechanical centripetal cutting method of a lens, the present disclosure mechanically and centripetally cuts the lens, and generates no thermal damage to intraocular tissues. The method is less prone to damaging the posterior capsule, zonules, and corneal endothelium, and features short operation duration, high efficiency, small surgical incisions, minimal discomfort, rapid recovery, ease to operate, and cost-efficiency, and widely applicable without increasing financial burden of the patient; it can create a small surgical incision without increasing a number of existing incisions, and it does not require ultrasound or laser, thereby avoiding various disadvantages of ultrasonic vibration.

Although the present disclosure has been disclosed as above in the form of preferred embodiments, it is not intended to limit the present disclosure. Those skilled in the art can make various modifications and variations without departing from the spirit and scope of the present disclosure. Therefore, the scope of protection of the present disclosure should be defined by the claims.

## Claims

1. A cataract surgical instrument, comprising:
a guide tube (3), wherein an end portion of the guide tube (3) is provided with an opening (31) in communication with an inner cavity (35) of the guide tube;
a deformation member (2), wherein the deformation member (2) comprises a deformation part (21) and a connecting part (22) that is connected to the deformation part (21), and the connecting part (22) is arranged through the inner cavity (35) and moves in an axial direction of the guide tube (3); and
a net bag (1), wherein the net bag (1) is connected to the deformation part (21); wherein
the connecting part (22) drives the deformation part (21) and the net bag (1) to move in the axial direction of the guide tube (3), such that the deformation part (21) and the net bag (1) retract into the inner cavity (35) or expand out of the inner cavity (35).

2. The cataract surgical instrument according to claim 1, further comprising a push rod (6), wherein the push rod (6) is connected to the connecting part (22), and is configured to drive the connecting part (22) to move in the axial direction of the guide tube (3).

3. The cataract surgical instrument according to claim 2, further comprising an outer tube (4), wherein the guide tube (3) is connected within the outer tube (4), and the push rod (6) is arranged through both the outer tube (4) and the guide tube (3), and is connected to the connecting part (22).

4. The cataract surgical instrument according to claim 3, wherein the guide tube (3) comprises a first tube section (32) and a second tube section (33) that is connected to the first tube section (32), the opening (31) is located at an end of the first tube section (32) away from the second tube section (33), an outer wall of the second tube section (33) is provided with at least one protrusion (34), an end portion of the outer tube (4) is provided with a slot (44) that matches the protrusion (34), and the guide tube (3) is assembled in the slot (44).

5. The cataract surgical instrument according to claim 4, wherein an interior of the outer tube (4) is sequentially provided with a first cavity (41), a second cavity (42), and a third cavity (43), a cross section of the second cavity (42) is smaller than cross sections of the first cavity (41) and the third cavity (43), and the guide tube (3) is assembled in the first cavity (41).

6. The cataract surgical instrument according to claim 5, wherein the push rod (6) comprises a first rod body (61) and a second rod body (62), a cross section of the first rod body (61) is smaller than a cross section of the second rod body (62), the second rod body (62) is arranged through the third cavity (43), and the first rod body (61) is arranged through the first cavity (41) and the second cavity (42), and is fixedly connected to the connecting part (22).

7. The cataract surgical instrument according to claim 6, wherein a push handle (63) is arranged at an end of the second rod body (62) away from the first rod body (61), and the push handle (63) is located outside the outer tube (4), and a plug (5) is disposed between the push handle and the outer tube (4).

8. The cataract surgical instrument according to any one of claims 1-7, wherein a size of the deformation part (21) is larger than a size of the inner cavity (35).

9. The cataract surgical instrument according to any one of claims 1-7, wherein the deformation part (21) is a ring structure formed by winding a long strip, the ring structure is an open-ring structure or a closed-ring structure, the long strip is made from a shape memory material, a height of the long strip is 0.01-2 mm, and a thickness of the long strip is 0.01-2 mm.

10. The cataract surgical instrument according to claim 9, wherein the net bag (1) is a bag-like structure with an aperture, the net bag is woven by a plurality of ultrafine filaments and formed with a plurality of mesh holes, a mesh diameter is 0.01-4 mm, and the aperture of the net bag (1) is connected to the long strip.
